# FASCICULE DE BREVET EUROPEEN

(11) **EP 1 140 932 B1**
(45) Date de publication et mention de la délivrance du brevet: **17.03.2004**
(21) Numéro de dépôt: 99961113.0
(22) Date de dépôt: 20.12.1999
(51) Int. Cl.: C07D 471/04, A61K 31/44, A61P 31/00

(54) **DERIVES DE LA BENZONAPHTYRIDINE-1,8**
BENZONAPHTYRIDIN-1,8 DERIVATE
1,8-BENZONAPHTHYRIDINE DERIVATIVES

(30) Priorité: 21.12.1998 FR 9816126; 11.06.1999 US 138582 P
(43) Date de publication de la demande: 10.10.2001
(73) Titulaire: Aventis Pharma S.A., 92160 Antony (FR)
(72) Inventeur: DESCONCLOIS, Jean-François, F-75012 Paris (FR); PICAUT, Guy, F-94800 Villejuif (FR); GIRARD, Philippe, Ollainville, F-91290 Arpajon (FR); TABART, Michel, F-91290 La Norville (FR); WENTZLER, Sylvie, F-94550 Chevilly Larue (FR)
(86) Numéro de dépôt international: PCT/FR1999/003206
(87) Numéro de publication internationale: WO 2000/037467

(56) Documents cités:
- EP-A- 0 379 412
- EP-A- 0 431 991
- EP-A- 0 536 035

## Description

La présente invention concerne de nouveaux dérivés de la benzo[b]naphtyridine-1,8 de formule générale : 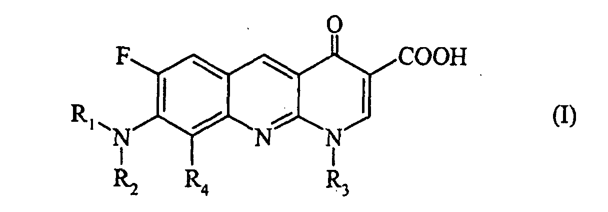 leurs sels, leur préparation et les compositions qui les contiennent.

Dans la demande de brevet EP 431 991 ont été décrits des dérivés de la benzonaphtyridine de structure : 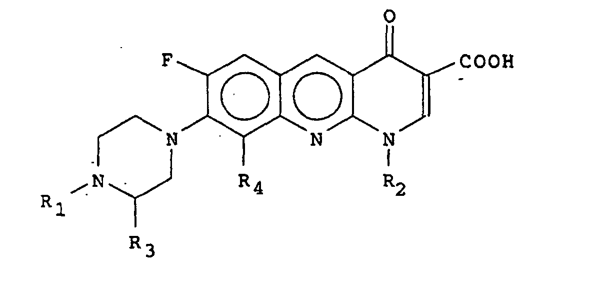 dans laquelle R₁ est H, hydroxy ou alcoyle, R₂ est H, alcoyle, fluoroalcoyle, cycloalcoyle, alcoyloxy ou alcoylamino, R₃ est phényle ou phénylalcoyle éventuellement substitué, et R₄ est H ou un atome de fluor. Ces produits sont utiles comme agents antimicrobiens.

Il a été trouvé maintenant que les dérivés de benzonaphtyridine de formule générale (I) dans laquelle :
- R₁ et R₂ identiques ou différents représentent un radical alcoyle ou cycloalcoyle contenant 3 à 8 chaînons, ou forment ensemble avec l'atome d'azote auquel ils sont attachés un hétérocycle à 6 chaînons contenant éventuellement un autre atome d'azote et pouvant être substitué en position -4 par un radical phényle, phényle substitué (par un atome d'halogène ou par un radical alcoylthiométhyle), benzyle, benzyle substitué par un atome d'halogène, hétérocyclylméthyle dont la partie hétérocyclyle est saturée ou insaturée et contient 5 ou 6 chaînons, phénylamino, ou phénylamino dont la partie phényle est éventuellement substituée par un atome d'halogène, ou R₁ et R₂ forment ensemble avec l'atome d'azote auquel ils sont attachés un hétérocycle à 7 ou 8 chaînons contenant éventuellement un autre atome d'azote et pouvant être substitué par l'un des substituants de l'hétérocycle à 6 chaînons énumérés ci-dessus,
- R₃ représente un radical alcoyle, fluoroalcoyle, carboxyalcoyle, cycloalcoyle contenant 3 à 6 atomes de carboné, fluorophényle, difluorophényle, alcoyloxy ou alcoylamino, et
- R₄ représente un atome d'hydrogène ou un atome de fluor,
les radicaux alcoyle cités ci-dessus étant droits ou ramifiés et contenant 1 à 4 atomes de carbone,
ainsi que leurs sels, et le cas échéant leurs stéréoisomères, manifestent une activité antibactérienne particulièrement intéressante pour la voie topique.

Dans la formule générale ci-dessus les substituants halogène peuvent être choisis parmi le chlore, le fluor, le brome ou l'iode, les radicaux hétérocyclyle peuvent être choisis notamment parmi furyle, thiényle, pyridyle, pyrimidyle, imidazolyle, thiazolyle, pyrrolidyle. Par ailleurs, lorsque R₁ et R₂ forment ensemble avec l'atome d'azote auquel ils sont attachés un hétérocycle à 6 à 8 chaînons, celui-ci peut être choisi à titre non limitatif parmi pipéridine, pipérazine, perhydroazépine, perhydroazocine, perhydrodiazépine, morpholine, thiomorpholine.

Selon l'invention, les produits de formule générale (I) peuvent être obtenus par substitution d'une amine

R₁-NH-R₂ (II)

dans laquelle R₁ et R₂ sont définis comme précédemment, sur une benzo[b]naphtyridine-1,8 de formule générale : 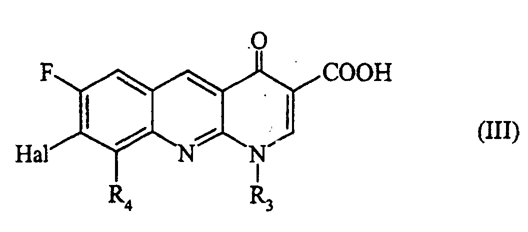 dans laquelle R₃ est défini comme précédemment, Hal est un atome de fluor, de chlore ou de brome si R₄ est hydrogène, ou bien Hal et R₄ sont simultanément des atomes de fluor.

L'action de l'amine de formule générale (II) s'effectue généralement en présence d'un excès de ce dérivé comme accepteur d'acide dans des solvants organiques convenables. Il est possible d'opérer avec ou sans solvant, à une température comprise entre 20 et 150°C. Lorsque l'on opère en présence d'un solvant, la réaction s'effectue avantageusement dans des solvants tels que la pyridine, le diméthylformamide, le diméthylsulfoxyde ou l'acétonitrile. Il est également possible d'opérer en milieu aqueux.

Il peut être également avantageux d'opérer en présence d'un accepteur d'acide comme par exemple une base organique azotée (triéthylamine notamment), un carbonate alcalin (carbonate de sodium par exemple) ou un hydroxyde de métal alcalin ou alcalinoterreux.

Selon l'invention, les dérivés de benzo[b]naphtyridine-1,8 de formule générale (I) peuvent être aussi obtenus à partir de l'ester correspondant de formule générale : 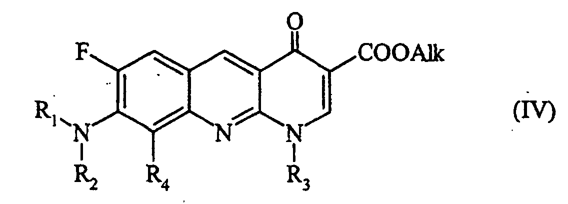 dans laquelle R₁, R₂ et R₄ sont définis comme précédemment, R₃ est défini comme précédemment ou représente un radical alcoylamino protégé et Alk représente un radical alcoyle contenant 1 à 4 atomes de carbone en chaîne droite ou ramifiée, par toute méthode connue pour obtenir un acide à partir d'un ester sans toucher au reste de la molécule.

La préparation de l'acide à partir de l'ester s'effectue généralement par saponification en présence de potasse ou de soude, en milieu aqueux ou hydro-alcoolique, à une température comprise entre 20 et 100°C ; il est également possible d'opérer par hydrolyse acide à des températures telles que citées ci-avant.

Lorsque R₃ représente un radical alcoylamino protégé, le radical protecteur peut être tout groupement protecteur d'amino compatible avec la molécule. Il est particulièrement avantageux de choisir un radical protecteur qui peut être éliminé simultanément à l'hydrolyse de l'ester. La protection peut être réalisée par tout groupement compatible et dont la mise en oeuvre et l'élimination n'altère pas le reste de la molécule. Notamment, on opère selon les méthodes décrites par T.W. GREENE, Protective Groups in Organic Synthesis, A. Wiley-Interscience Publication (1981), ou par Mc OMIE, Protective Groups in Organic Chemistry, Plenum Press (1973).

Le dérivé de la benzo[b]napthyridine-1,8 de formule générale (III) peut être obtenu par application des méthodes décrites dans le brevet US 4 990 515, EP 431 991 ou EP 379 414 ou par analogie avec ces techniques.

Le dérivé de la benzo[b]napthyridine-1,8 de formule générale (IV) peut être obtenu par action de l'amine de formule générale (II) sur l'ester correspondant de formule générale : 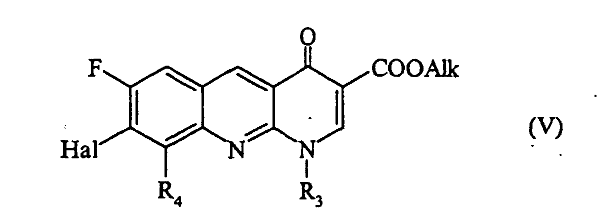 dans laquelle R₃, R₄, Hal et Alk sont définis comme précédemment, selon la méthode décrite pour la réaction de l'amine de formule générale (II) avec le dérivé de benzonaphtyridine de formule générale (III). Il est entendu que dans l'alternative oùl'on opère en milieu aqueux il est possible d'obtenir directement le produit de formule générale (I) sans isoler intermédiairement le dérivé de formule générale (IV).

L'ester dérivé de la benzo[b]naphtyridine-1,8 de formule générale (V) peut être obtenu comme décrit dans le brevet européen EP 606 382.

Selon l'invention, le cas échéant lorsque des formes stéréoisomères des dérivés de la benzonaphtyridine de formule générale (I) existent et lorsque l'on veut obtenir ces stéréoisomères, la séparation des formes stéréoisomères des amines de formule générale (II) est effectuée, par toute méthode connue et compatible avec la molécule. A titre d'exemple, la séparation s'effectue par acylation au moyen d'un acide ou d'un dérivé réactif d'un acide chiral, séparation des isomères par chromatographie liquide hautes performances, puis désacylation selon la méthode décrite par P. G. Gasseman et coll., J. Am. Chem. Soc., 98 (5), 1275 (1976). Il est également possible d'effectuer la séparation des stéréoisomères par chromatographie liquide hautes performances sur phase chirale.

Les nouveaux produits selon la présente invention ainsi que leurs intermédiaires de synthèse peuvent être éventuellement purifiés par des méthodes physiques telles que la cristallisation ou la chromatographie.

Les produits selon la présente invention ainsi que leurs intermédiaires de formule générale (III) peuvent être transformés en sels métalliques ou en sels d'addition avec les bases azotées selon les méthodes connues en soi. Ces sels peuvent être obtenus selon les méthodes habituelles qui n'altèrent pas le reste de la molécule, notamment par action d'une base métallique (par exemple alcaline ou alcalino-terreuse), de l'ammoniac ou d'une amine sur un produit cité ci-dessus dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Les nouveaux produits selon l'invention peuvent être également transformés en sels d'addition avec les acides. Les produits de formule générale (I) obtenus sous forme de ces sels, peuvent être libérés et transformés en sels d'autres acides selon les méthodes habituelles.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylaminé, triéthylamine, méthylamine, propylamine, diisopropyl-amine, NN-diméthyléthanolamine, benzylamine, dicyclohexylamine, N-benzyl phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonates, iséthionates).

Les nouveaux dérivés de benzo[b]naphtyridine-1,8 de formule générale (I) selon la présente invention et leurs sels pharmaceutiquement acceptables présentent des propriétés antibactériennes particulièrement intéressantes. Ils manifestent une activité remarquable in vitro et in vivo sur les germes gram-positifs et plus particulièrement sur les germes résistants aux quinolones. Compte tenu de leur activité, ils sont tout particulièrement indiqués pour l'usage par voie topique.

In vitro, les produits de formule générale (I) se sont montrés actifs à une concentration comprise entre 0,06 et 4 µg/cm3 sur staphylococcus aureus IP 8203 et à une concentration comprise entre 0,12 et 4 µg/cm3 sur staphylococcus aureus N79 résistant aux quinolones.

In vivo, le produit de l'exemple 1 s'est montré actif à une concentration de 2 % dans une formulation de cetomacrogol et d'alcool benzylique, dans le modèle de contamination du cobaye à staphylococcus aureus ATCC 25923. Il s'est également montré actif à 2 % sur le modèle d'infection expérimentale du cobaye à staphylococcus aureus AS 5155 souche résistante à la méticilline, au MLS_{B} et aux quinolones.

Enfin les produits selon l'invention ne présentent pas de toxicité aux doses utilisées. Après 2 semaines d'application topique b.i.d. chez le rat d'une composition comprenant le produit de l'exemple 1 à raison de 2, 5 et 10 %, aucun effet toxique n'a été observé. De même, après 2 semaines d'application topique b.i.d. rat d'une composition comprenant le produit de l'exemple 1 à raison de 2 %, sur la muqueuse nasale du lapin aucun effet toxique n'a été non plus observé.

D'un intérêt particulier sont les produits de formule générale (I) pour lesquels :
- R₁ et R₂ identiques ou différents représentent un radical alcoyle contenant 1 ou 2 atomes de carbone, ou cyclopropyle, ou forment ensemble avec l'atome d'azote auquel ils sont attachés un hétérocycle à 6 chaînons, contenant éventuellement un autre atome d'azote et pouvant être substitué en position -4 par un radical phényle, phényle substitué par un atome d'halogène ou par un radical méthylthiométhyle, benzyle, benzyle substitué par un atome d'halogène, hétérocyclylméthyle dont la partie hétérocyclyle est insaturée et contient 5 chaînons, phénylamino dont la partie phényle est éventuellement substituée par un atome d'halogène, ou R₁ et R₂ forment ensemble avec l'atome d'azote auquel ils sont attachés un hétérocycle à 7 ou 8 chaînons,
- R₃ représente un radical alcoyle contenant 1 ou 2 atomes de carbone, cyclopropyle, difluorophényle ou méthylamino, et
- R₄ représente un atome d'hydrogène ou un atome de fluor
ainsi que leurs sels ;
et parmi ces produits plus particulièrement l'acide-7-fluoro-8-[4-(4-fluorophényl)-pipérazin-1-yl]-1-méthyl-4-oxo-1,4-dihydro-benzo[b][1,8]naphtyridine-3-carboxylique et ses sels.

Les exemples suivants donnés à titre non limitatif illustrent la présente invention.

### Exemple 1

### Acide-7-fluoro-8-[4-(4-fluorophényl)-pipérazin-1-yl]-1-methyl-4-oxo-1,4-dihydrobenzo[b][1,8]naphtyridine-3-carboxylique :

Une suspension de 5,0 g de l'acide-7,8-difluoro-1-méthyl-4-oxo-1,4-dihydrobenzo[b][1,8]naphtyridine-3-carboxylique et de 6,2 g de 4-(4-fluorophényl) pipérazine dans 30 cm³ de diméthylsulfoxyde est chauffée, sous agitation, à une température voisine de 90°C pendant environ 4 heures. Après refroidissement à environ 20°C, on ajoute au mélange réactionnel 25 cm³ d'eau. L'insoluble est-essoré et lavé par 2 fois avec 25 cm³ d'eau.Après 1 recristallisation dans 150 cm³ de diméthylformamide, on obtient 7,6 g d' acide 7-fluoro-8-[-4-(-4-fluorophényl)-pipérazin-1-yl]-1-méthyl-4-oxo-1,4-dihydro-benzo[b][1,8]naphtyridine-3-carboxy-lique sous forme d'un solide jaune fondant à 318-9°C.

### 7-Fluoro-8-[4-(4-fluorophényl)-pipérazin-1-yl]-1-méthyl-4-oxo-1,4-dihydrobenzo[b][1,8]naphtyridine-3-carboxylate de choline :

A une suspension de 7,6 g d'acide 7-fluoro-8-[4-(4-fluorophényl)-pipérazin-1-yl]-1-méthyl-4-oxo-1,4-dihydro-benzo[b][1,8]naphtyridine-3-carboxylique dans 75 cm³ de méthanol sont ajoutés 7,6 cm³ d'une solution d'hydroxyde de choline à 45% dans le méthanol. Le mélange réactionnel est agité environ 2 heures vers 25°C. La solution est filtrée, si nécessaire, pour éliminer un léger insoluble et concentrée vers 50°C à l' évaporateur rotatif sous environ 20 kPa. Le solide résiduel est recristallisé dans 70 cm³ de 2 propanol et 30 cm³ de méthanol. Les cristaux sont essorés avec 10 cm³ de 2-propanol à chaque fois et séchés à l'étuve à 100°C, sous 10 kPa On obtient 7,4 g de 7-fluoro-8-[4-(4-fluorophényl)-pipérazin-1-yl]-1-méthyl-4-oxo-1,4-dihydrobenzo[b][1,8]naphtyridine-3-carboxylate de choline sous forme d'un solide jaune fondant à 285-90°C.

L'acide 7,8-difluoro-1-méthyl-4-oxo-1,4-dihydro-benzo[b][1,8]naphtyridine-3-carboxylique a été préparé selon la méthode décrite dans le brevet EP 431 991.

### Exemple 2

### Acide-7-fluoro-8-[4-(4-fluorophényl)-pipérazin-1-yl]-1-méthyl-4-oxo-1,4-dihydrobenzo[b][1,8]naphtyridine-3-carboxylique :

Une suspension de 3,07 g d'acide 8-chloro-7-fluoro-1-méthyl-4-oxo-1,4-dihydrobenzo[[b][1,8]naphtyridine-3-carboxylique et de 7,21 g de 4-(4-fluorophényl) pipérazine dans 30 cm³ de pyridine est chauffée à une température voisine de 115°C pendant 84 heures. Le mélange réactionnel est concentré sous pression réduite (20kPa) à environ 60°C. Le solide obtenu est repris par 50 cm³ d'eau et additionné de 3,5 cm³ d' acide acétique à 10%. La suspension est portée vers 100°C. Le produit concrétisé est essoré, lavé par 3 fois avec 30 cm³ d'eau et 3 fois 30 cm³ d'éthanol chaud. Après 2 recristallisations dans 65 cm³ de diméthylformamide à chaque fois, on obtient 2,82 g d'acide-7-fluoro-8-[4-(4-fluorophényl)-pipérazin-1-yl]-1-méthyl-4-oxo-1,4-dihydro-benzo[b][1,8]naphtyridine-3-carboxylique sous forme d'un solide jaune fondant à 318-319°C.

L'acide 8-chloro-7-fluoro-1-méthyl-4-oxo-1,4-dihydro-benzo[b][1,8]naphtyridine-3-carboxylique a été préparé dans les conditions décrites dans le brevet EP 431991.

### Exemple 3

### Acide-7-fluoro-8-[4-(4-fluorophényl)-pipérazin-1-yl]-1-méthyl-4-oxo-1,4-dihydrobenzo[b][1,8]naphtyridine-3-carboxylique :

Une suspension de 10,5 g de 7-fluoro-8-[4-(4-fluorophényl)-pipérazin-1-yl]-1-méthyl-4-oxo-1,4-dihydro-benzo[b][1,8]naphtyridine-3-carboxylate d'éthyle dans 100 cm³ d'acide acétique et 100 cm³ d' acide chlorhydrique en solution aqueuse à 17,5% est chauffée, sous agitation, à une température voisine de 110°C pendant environ 4 heures. Après refroidissement à environ 20°C, on ajoute au mélange réactionnel 25 cm³ d'eau. L'insoluble est essoré et lavé par 3 fois avec 200cm³ d'eau et par 2 fois avec 100cm³ d'éthanol. On obtient 9,3g d'acide-7-fluoro-8-[4-(4-fluorophényl)-pipérazin-1-yl]-1-méthyl-4-oxo-1,4-dihydro-benzo[b][1,8]naphty-ridine-3-carboxylique sous forme d'un solide jaune fondant vers 320°C, qui est utilisé sans autre purification pour les étapes ultérieures.

Le 7-fluoro-8-[4-(4-fluorophényl)-pipérazin-1-yl]-1-méthyl-4-oxo-1,4-dihydrobenzo[b][1,8]naphtyridine-3-carboxylate d'éthyle est préparé par analogie avec la méthode décrite dans l'exemple 1 à partir de 3,2 g de 7,8-difluoro-1-méthyl-4-oxo-1,4-dihydro-benzo[b][1,8]naphtyridine-3-carboxylate d'éthyle, de 2,16 g de 4-(4-fluorophényl) pipérazine et de 3,35 cm³ de triéthylamine dans 40 cm³ de diméthylsulfoxyde. Après un essorage avec 15 cm³ d'eau par 2 fois et 15 cm³ d'éthanol par 2 fois on obtient 0,4 g de 7-fluoro-8-[4-(4-fluorophényl)-pipérazin-1-yl]-1-méthyl-4-oxo-1,4-dihydro-benzo[b][1,8]naphtyridine-3-carboxylate d'éthyle sous forme d'un solide jaune fondant à 310°C

Le 7,8-difluoro-1-méthyl-4-oxo-1,4-dihydro-benzo[b][1,8]naphtyridine-3-carboxylate d'éthyle est préparé comme décrit dans le brevet EP 431 991.

### Exemple 4

### Acide 8-(cyclohexyl méthyl amino)-7-fluoro-1-méthyl-4-oxo-1,4-dihydro-benzo[b] [1,8]naphtyridine-3-carboxylique :

L'acide 8-(cyclohexyl méthyl amino)-7-fluoro-1-méthyl-4-oxo-1,4-dihydro-benzo[b] [1,8]naphtyridine-3-carboxylique a été préparé dans les conditions de l'exemple 1, mais à partir de 1,5 g d'acide-7,8-difluoro-1-méthyl-4-oxo-1,4-dihydro-benzo[b] [1,8]naphtyridine-3-carboxylique et de 1,3 cm³ de N-méthylcyclohexylamine dans 30 cm³ de diméthylsulfoxyde. Après un lavage avec 15 cm³ d'éthanol par 2 fois on obtient 1,7 g d'acide 8-(cyclohexyl méthyl amino)-7-fluoro-1-méthyl-4-oxo-1,4-dihydrobenzo[b][1,8]naphtyridine-3-carboxylique sous forme d'un solide jaune fondant à 265°C.

### 8-(Cyclohexyl méthyl amino)-7-fluoro-1-méthyl-4-oxo-1,4-dihydro-benzo[b][1,8] naphtyridine-3-carboxylate de choline

Le 8-(cyclohexyl méthyl amino)-7-fluoro-1-méthyl-4-oxo-1,4-dihydro-benzo[b][1,8] naphtyridine-3-carboxylate de choline a été préparé dans les conditions de l'exemple 1, mais à partir 1,5 g d'acide 8-(cyclohexyl méthyl amino)-7-fluoro-1-méthyl-4-oxo-1,4-dihydro-benzo[b][1,8]naphtyridine-3-carboxylique dans 15 cm³ de méthanol et 1,5 cm³ d'une solution d'hydroxyde de choline à 45% dans le méthanol. Après recristallisation dans 15 cm³ de 2 propanol, on obtient le 8-(cyclohexyl méthyl amino)-7-fluoro-1-méthyl-4-oxo-1,4-dihydro-benzo[b][1,8] naphtyridine-3-carboxylate de choline sous forme d'un solide jaune fondant à 245-250°C.

### Exemple 5

### Acide 8-azocan-1-yl-7-fluoro-1-méthyl-4-oxo-1,4-dihydro-benzo[b][1,8] naphtyridine-3-carboxylique :

L'acide 8-azocan-1-yl-7-fluoro-1-méthyl-4-oxo-1,4-dihydro-benzo[b][1,8]naphtyridine-3-carboxylique est préparé dans les conditions de l'exemple 1, mais à partir de 1 g d'acide-7,8-difluoro-1-méthyl-4-oxo-1,4-dihydro-benzo[b][1,8]naphtyridine-3-carboxylique et de 0,87 cm³ de heptaméthylèneimine dans 20 cm³ de diméthylsulfoxyde. Après un lavage avec 15 cm³ d'éthanol par 2 fois on obtient 1,1 g d'acide 8-azocan-1-yl-7-fluoro-1-méthyl-4-oxo-1,4-dihydro-benzo[b][1,8]naphtyridine-3-carboxylique sous forme d'un solide jaune fondant à 320-325°C.

### Exemple 6

### Acide 7-fluoro-1-méthyl-8-[4-(3-méthylsulfanylméthyl-phényl)-pipérazin-1-yl]-4-oxo-1,4-dihydro-benzo[b][1,8]naphtyridine-3-carboxylique :

L'acide 7-fluoro-1-méthyl-8-[4-(3-méthylsulfanylméthyl-phényl)-pipérazin-1-yl]-4-oxo-1,4-dihydro-benzo[b][1,8]naphtyridine-3-carboxylique a été préparé dans les conditions de l'exemple 1, mais à partir de 0,45 g d'acide-7,8-difluoro-1-méthyl-4-oxo-1,4-dihydro-benzo[b][1,8]naphtyridine-3-carboxylique et de 0,66g de (3-méthylsulfanylméthyl-phényl)-pipérazine dans 15 cm³ de diméthylsulfoxyde. Après un lavage avec 15 cm³ d'éthanol par 2 fois on obtient 0,61 g d'acide 7-fluoro-1-méthyl-8-[4-(3-méthylsulfanylméthyl-phényl)-pipérazin-1-yl]-4-oxo-1,4-dihydro-benzo[b][1,8] naphtyridine-3-carboxylique sous forme d'un solide jaune fondant à 240-5°C.

La (3-méthylsulfanylméthyl-phényl)-pipérazine est préparée selon la méthode suivante:
On chauffe à 90°C, sous un courant d'azote, pendant 24 heures un mélange de 2,5 g de 1-iodo-3-méthylsulfanylméthyl-benzène, 1,3 g de tertiobutylate de sodium, 0,35 mg de chlorure de 1,1'-bis(diphénylphosphino)ferrocényl palladium, 0,79 g de 1,1'-bis(diphénylphosphino)ferrocène, 4g de pipérazine et 100 cm³ de toluène. Le milieu réactionnel est refroidi à température ambiante et filtré sur verre fritté. Le filtrat est lavé par 250 cm³ de dichlorométhane puis concentré à sec sous pression réduite (2,7 kPa). Le résidu obtenu est purifié par chromatographie sur colonne de gel de silice (granulométrie 0,04-0,06 mm, diamètre 4 cm, hauteur 20 cm) sous une pression de 0,5 bar d'azote avec un mélange de dichlorométhane et de méthanol (90/10 en volumes) puis un mélange de dichlorométhane et de méthanol (85/15 en volumes) comme éluants et en recueillant des fractions de 100 cm³. Les fractions 29 à 36 sont réunies, concentrées à sec sous pression réduite (2,7 kPa). L'huile obtenue est distillée au four à boule (Eb=160°C environ sous une pression de 0,1 mm de mercure). On obtient 0,67 g de 4-(3-méthylsulfanylméthyl-phényl)-pipérazine sous forme d'une huile incolore.

### Exemple 7

### Acide 7-fluoro-8-[-4-(-4-fluoroanilino)-pipéridino]-1-méthyl-4-oxo-1,4-dihydro-benzo[b][1,8]naphtyridine-3-carboxylique :

L'acide 7-fluoro-8-[-4-(-4-fluoroanilino)-pipéridino]-1-méthyl-4-oxo-1,4-dihydro-benzo[b][1,8]naphtyridine-3-carboxylique a été préparé dans les conditions de l'exemple 2, mais à partir de 1,6 g d'acide 8-chloro 7-fluoro-1-méthyl-4-oxo-1,4-dihydro-benzo[b][1,8]naphtyridine-3-carboxylique et de 3,7 g de 4-(4-fluoroanilino) pipéridine dans 16 cm³ de pyridine. Après recristallisation dans 10 cm³ de diméthylformamide, on obtient 1,65 g d'acide 7-fluoro-8-[4-(4-fluoroanilino)-pipéridino]-1-méthyl-4-oxo-1,4-dihydro-benzo[b][1,8]naphtyridine-3-carboxylique sous forme- d'un solide jaune fondant à 280°C.

### 7-Fluoro-8-[-4-(-4-fluoroanilino)-pipéridino]-1-méthyl-4-oxo-1,4-dihydro-benzo[b] [1,8]naphtyridine-3-carboxylate de choline

Le-7-fluoro-8[-4-(-4-fluoroanilino)-pipéridino]-1-méthyl-4-oxo-1,4-dihydro-benzo[b] [1,8]naphtyridine-3-carboxylate de choline a été préparé dans les conditions de l'exemple 1, mais à partir de 1,6 g d'acide 7-fluoro-8-[-4-(4-fluoroanilino)-pipéridino]-1-méthyl-4-oxo-1,4-dihydro-benzo[b][1,8]naphtyridine-3-carboxylique dans 16 cm³ de méthanol et 1,3 cm³ d'une solution d'hydroxyde de choline à 45 % dans le méthanol.

Après 2 recristallisations dans 25 cm³ de 2-propanol à chaque fois, on obtient 1,1 g de 7-fluoro-8-[4-(4-fluoroanilino)-pipéndino]-1-méthyl-4-oxo-1,4-dihydro-benzo[b][1,8]naphtyridine-3-carboxylate de choline sous forme d'un solide jaune fondant à 200-205°C.

La 4-(4-fluoroanilino) pipéridine a été préparée selon le brevet US 3,686,187.

### Exemple 8

### Acide 7-fluoro-8-[4-(-4-fluorobenzyl)-pipérazin-1-yl]-1-méthyl-4-oxo-1,4-dihydro-benzo[b][1,8]naphtyridine-3-carboxylique :

L'acide 7-fluoro-8-[4-(-4-fluorobenzyl)-pipérazin-1-yl]-1-méthyl-4-oxo-1,4-dihydro-benzo[b][1,8]naphtyridine-3-carboxylique a été préparé dans les conditions de l'exemple 2, mais à partir de 1,6 g d'acide 8-chloro-7-fluoro-1-méthyl-4-oxo-1,4-dihydro-benzo[b][1,8]naphtyridine-3-carboxylique et de 5 g de 4-(4-fluorobenzyl) pipérazine dans 16 cm³ de pyridine. Après 2 recristallisations dans 25 cm³ de diméthylformamide à chaque fois,on obtient 1,4 g d'acide-7-fluoro-8-[4-(-4-fluorobenzyl)-pipérazin-1-yl]-1-méthyl-4-oxo-1,4-dihydro-benzo[b][1,8]naphtyri-dine-3-carboxylique sous forme d'un solide jaune fondant à 311°C.

### Exemple 9

### Acide 7-fluoro-8-[4-benzyl-pipérazin-1-yl]-1-méthyl-4-oxo-1,4-dihydro-benzo[b] [1,8]naphtyridine-3-carboxylique :

L'acide 7-fluoro-8-[4-benzyl-pipérazin-1-yl]-1-méthyl-4-oxo-1,4-dihydro-benzo[b] [1,8]naphtyridine-3-carboxylique a été préparé dans les conditions de l'exemple 2, mais à partir de 2 g d'acide 8-chloro-7-fluoro-1-méthyl-4-oxo-1,4-dihydro-benzo[b][1,8]naphtyridine-3-carboxylique et de 4,6 g de N-benzylpipérazine dans 20 cm³ de pyridine. Après 2 recristallisations dans 25 cm³ de diméthylformamide à chaque fois, on obtient 1,4 g d' acide 7-fluoro-8-[4-benzyl-pipérazin-1-yl]-1-méthyl-4-oxo-1,4-dihydro-benzo[b][1,8]naphtyridine-3-carboxylique sous forme d'un solide jaune fondant à 311°C.

### Exemple 10

### Acide 1-éthyl-7-fluoro-8-[-4-(-4-fluorophényl)-pipérazin-1-yl]-4-oxo-1,4-dihydro-benzo[b][1,8]naphtyridine-3-carboxylique

L'acide 1-éthyl-7-fluoro-8-[-4-(-4-fluorophényl)-pipérazin-1-yl]-4-oxo-1,4-dihydro-benzo[b][1,8]naphtyridine-3-carboxylique a été préparé dans les conditions de l'exemple 2, mais à partir de 1,75 g d'acide 8-chloro-1-éthyl-7-fluoro-4-oxo-1,4-dihydro-benzo[b][1,8]naphtyridine-3-carboxylique et de 3,6 g de 4-(4-fluorophényl) pipérazine dans 18 cm³ de pyridine. Le solide obtenu est repris par 10 cm³ d'eau et additionné de 1,9 cm³ d' acide acétique à 10%. La suspension est portée vers 100°C. Le produit concrétisé est essoré, lavé par 2 fois avec 10 cm³ d'eau et 2 fois 10 cm3 d'éthanol chaud. Après 2 recristallisations dans 25 cm³ de diméthylformamide à chaque fois, on obtient 1,7 g d'acide 1-éthyl-7-fluoro-8-[4-(-4-fluorophényl)-pipérazin-1-yl]-4-oxo-1,4-dihydro-benzo[b][1,8]naphtyridine-3-carboxylique-3 sous forme d'un solide beige rosé fondant à 311-312°C.

### Exemple 11

### Acide 1-éthyl-7-fluoro-8-[-4-(-4-fluorobenzyl)-pipérazin-1-yl]-4-oxo-1,4-dihydro-benzo[b][1,8]naphtyridine-3-carboxylique

L'acide 1-éthyl-7-fluoro-8-[-4-(-4-fluorobenzyl)-pipérazin-1-yl]-4-oxo-1,4-dihydro-benzo[b][1,8]naphtyridine-3-carboxylique a été préparé dans les conditions de l'exemple 2, mais à partir de 1,6 g d'acide 8-chloro-1-éthyl 7-fluoro-4-oxo-1,4-dihydro-benzo[b][1,8]naphtyridine-3-carboxylique et de 3,9 g de 4-(4-fluorobenzyl) pipérazine. Après 2 recristallisations dans 20 cm³ de diméthylformamide à chaque fois, on obtient 1,4 g d'acide 1-éthyl-7-fluoro-8-[4-(-4-fluorobenzyl)-pipérazin-1-yl]-4-oxo-1,4-dihydrobenzo[b][1,8]naphtyridine-3-carboxylique sous forme d'un solide jaune fondant à 286-8°C.

### Exemple 12

### Acide 1-cyclopropyl-7,9-difluoro-8-(-4-furfuryl-pipérazin-1-yl)-4-oxo-1,4-dihydro-benzo[b][1,8]naphtyridine-3-carboxylique :

Une suspension de 1,5 g de 1-cyclopropyl-7,9-difluoro-8-(-4-furfuryl-pipérazin-1-yl)-4-oxo-1,4-dihydro-benzo[b][1,8]naphtyridine-3-carboxylate d'éthyle est chauffée, sous agitation, à une température voisine de 80°C pendant environ 1 heure 30 dans 15 cm³ d'éthanol et 14 cm³ de potasse 1 N, additionnée à cette même température de 32 cm3 d'acide acétique à 5% et agitée pendant 20 minutes. Après une recristallisation dans un mélange de 15 cm³ de diméthylformamide et de 15 cm³ d'éthanol, on obtient 0,7 g d'acide 1-cyclopropyl-7,9-difluoro-8-(4-furfuryl-pipérazin-1-yl)-4-oxo-1,4-dihydro-benzo[b][1,8]naphtyridine-3-carboxylique sous forme d'un solide jaune fondant à 241°C.

Le 1-cyclopropyl-7,9-difluoro-8-(4-furfuryl-pipérazin-1-yl)-4-oxo-1,4-dihydro-benzo[b][1,8]naphtyridine-3-carboxylate d'éthyle est préparé dans les conditions de l'exemple 3, mais à partir de 1,8 g de 1-cyclopropyl-7,8,9-trifluoro-4-oxo-1,4-dihydrobénzo[b][1,8]naphtyridine-3-carboxylate d'éthyle et de 4,8 g du chlorhydrate de 4-furfurylpipérazine dans 30 cm³ de diméthylsulfoxyde et 5,2 cm³ de triéthylamine. On obtient 1,5 g de 1-cyclopropyl-7,9-difluoro-8-(4-furfuryl-pipérazin-1-yl)-4-oxo-1,4-dihydro-benzo[b][1,8]naphtyridine-3-carboxylate d'éthyle sous forme d'un solide jaune brun fondant à 125-128°C qui est utilisé sans autre purification pour les étapes ultérieures.

Le 1-cyclopropyl-7,8,9-trifluoro-4-oxo-1,4-dihydrobenzo[b][1,8]naphtyridine-3-carboxylate d'éthyle peut être préparé comme décrit dans le brevet EP 431 991.

Le chlorhydrate de 4-furfurylpipérazine a été préparé selon la méthode décrite par M. Pesson et coll., Eur. J. Med. Chim. Ther., 10, 567-570 ( 1975 ).

### Exemple 13

### Acide 7,9-difluoro-1-(2,4-difluorophényl)-8-[4-(4-fluorophényl)-pipérazin-1-yl]-4-oxo-1,4-dihydro-benzo[b][1,8]naphtyridine-3-carboxylique :

Une suspension de 1,2 g de 7,9-difluoro-1-(2,4-difluorophényl)-8-[4-(4-fluorophényl)-pipérazin-1-yl]-4-oxo-1,4-dihydro-benzo[b]-[1,8]naphtyridine-3-carboxylate d'éthyle dans 15 cm³ d'éthanol et 16 cm3 de potasse aqueuse 1N est chauffée sous agitation à une température voisine de 80°C pendant 2 heures. La solution obtenue est additionnée de 10 cm³ d'une solution aqueuse d'acide acétique à 10%, à environ 80°C. L'insoluble est essoré à cette température, lavé par 2 fois 30 cm3 d'eau et recristallisé dans un mélange de 12,5 cm³ d'éthanol et 12,5 cm³ de diméthylformamide. On obtient - 0,75 g d'acide 7,9-difluoro-1-(2,4-difluorophényl)-8-[4-(4-fluorophényl)-pipérazin-1-yl]-4-oxo-1,4-dihydro-benzo[b][1,8]naphtyridine-3-carboxylique sous forme d'un solide jaune fondant à 284-286°C.

Le 7,9-difluoro-1-(2,4-difluorophényl)-8-[4-(4-fluorophényl)-pipérazin-1-yl]-4-oxo-1,4-dihydro-benzo[b][1,8]naphtyridine-3-carboxylate d'éthyle est préparé dans les conditions suivantes :
une suspension de 1,5 g de-1-(2,4-difluorophényl)-7,8,9-trifluoro-4-oxo-1,4-dihydro-benzo[b][1,8]naphtyridine-3-carboxylate d'éthyle et de 1,8 g de 4-(4-fluorophényl)-pipérazine dans 20 cm³ de diméthylsulfoxyde est chauffée, sous agitation, à une température voisine de 100°C pendant environ 1 heure. Après refroidissement vers 20°C, le mélange réactionnel est versé dans 100 cm³ d'eau et extrait par 3 fois 50 cm³ de trichlorométhane. Les extraits organiques réunis sont lavés par 1 fois 50 cm3 d'eau, séchés sur sulfate de magnésium et concentrés sous pression réduite (20 kPa) à une température voisine de 40°C. On obtient 1,7 g de 7,9-difluoro-1-(2,4-difluorophényl)-8-[4-(4-fluorophényl)-pipérazin-1-yl]-4-oxo-1,4-dihydro-benzo[b][1,8] naphtyridine-3-carboxylate d'éthyle sous forme d'un solide jaune fondant à 298-299°C qui est utilisé sans autre purification pour les étapes ultérieures.

La 1-(2,4-difluorophényl)-7,8,9-trifluoro-4-oxo-1,4-dihydro-benzo[b][1,8]naphtyridine-3-carboxylate d'éthyle est préparée de la maniére suivante :
dans une solution de 7,7 g de 3-(2-chloro-6,7,8-trifluoro-3-quinolyl)-2-diméthylaminométhylène-3-oxo-propionate d'éthyle dans 50 cm³ de trichlorométhane, on ajoute à une température voisine de 20°C, 5,2 g de difluoro 2,4 aniline et laisse sous agitation pendant 8 heures à cette température. Le trichlorométhane est concentré sous pression réduite ( 20 kPa ) à une température voisine de 40°C Le mélange est additionné de 120 cm³ d'éthanol et de 4,5 cm³ de 1,8-diazabicyclo[5.4.0]undec-7-ène et chauffé à une température voisine de 80°C pendant environ 1 heure. Après refroidissement à une température voisine de 10°C, le produit est essoré, lavé par 2 fois 50 cm³ d'éthanol et 2 fois 50 cm³ d'éther isopropylique. On obtient 5 g de 1-(2,4-difluorophényl)-7,8,9-trifluoro-4-oxo-1,4-dihydro-benzo[b][1,8]naphtyridine-3-carboxylate d'éthyle sous forme d'un solide jaune fondant à 304-306°C qui est utilisé sans autre purification pour les étapes ultérieures.

Le 3-(2-chloro-6,7,8-trifluoro-3-quinolyl)-2-diméthylaminométhylène-3-oxo-propionate d'éthyle est décrit dans le brevet EP 431 991.

### Exemple 14

### Acide 7-fluoro-8-[4-(4-fluorophényl)-pipérazin-1-yl]-1-méthylamino-4-oxo-1,4-dihydro-benzo[b]-[1,8]naphtyridine-3-carboxylique.

L'acide 7-fluoro-8-[4-(4-fluorophényl)-pipérazin-1-yl]-1-méthylamino-4-oxo-1,4-dihydro-benzo[b][1,8]-naphtyridine-3-carboxylique est préparé dans les conditions de l'exemple 2, mais à partir de 3,2 g d'acide 8-chloro-7-fluoro-1-méthylamino-4-oxo-1,4-dihydro-benzo[b][1,8]naphtyridine-3-carboxylique et de 9 g de 4-(4-fluorophényl) pipérazine dans 30 cm³ de pyridine pendant 28 heures Après concentration à sec du mélange réactionnel sous pression réduite (20 kPa) vers 60°C, le résidu est repris 2 fois par 30 cm³ d'éthanol et concentré sous pression réduite dans les conditions ci-dessus. Le solide est repris par 50cm³ d'eau et 10 cm³ de potasse aqueuse à 30 %. Le précipité formé est additionné de 20 cm³ d'eau et d'environ 10 cm3 d'acide acétique à 10 %. La suspension est extraite par 3 fois 50 cm³ de trichlorométhane. Le trichlorométhane est concentré sous pression réduite (20kPa) et le solide obtenu est repris avec 2 fois 20 cm³ d'éthanol. Après 1 cristallisation dans 42 cm³, puis 1 recristallisation dans 15 cm³ de diméthylformamide on obtient 1,33 g d'acide 7-fluoro-8-[4-(4-fluorophényl)-pipérazin-1-yl]-1-méthylamino-4-oxo-1,4-dihydro-benzo[b][1,8]-naphtyridine-3-carboxylique sous forme d'un solide brun fondant à 304-305°C.

L'acide 8-chloro-7-fluoro-1-méthylamino-4-oxo-1,4-dihydro-benzo[b][1,8]naphtyridine-3-carboxylique est décrit dans le brevet EP 379 414.

### Exemple 15

### Acide-1-cyclopropyl-7-fluoro-8-[4-(4-fluoroanilino)-pipéridino]-4-oxo-1,4-dihydro-benzo[b][1,8]naphtyridine-3-carboxylique :

Une suspension de 1,2 g de 1-cyclopropyl-7-fluoro-8-[4-(-4-fluoroanilino)-pipéridino-4-oxo-1,4-dihydro-benzo[b][1,8]naphtyridine-3-carboxylate d'éthyle dans 20 cm³ d'acide acétique et 20 cm³ d'acide chlorhydrique en solution aqueuse à 17,5 % est chauffée sous agitation à une température voisine de 110°C pendant 4 heures. Après refroidissement à environ 20°C, la solution est additionnée de 30 cm³ d'une solution aqueuse d' hydroxyde de sodium à 15%. L'insoluble est essoré, lavé par 2 fois 30 cm³ d'eau et recristallisé dans un mélange de 50 cm³ d'éthanol et 60 cm³ de diméthylformamide. On obtient 1 g d'acide 1-cyclopropyl-7-fluoro-8-[4-(-4-fluoroanilino)-pipéridino]-4-oxo-1,4-dihydro-benzo[b][1,8]naphtyridine-3-carboxylique sous forme d'un solide jaune fondant à 295°C.

Le 1-cyclopropyl-7-fluoro-8-[4-(-4-fluoroanilino)-pipéridino-4-oxo-1,4-dihydro-benzo[b][1,8]naphtyridine-3-carboxylate d'éthyle est préparé dans les conditions suivantes : une suspension de 2,8 g de 8-chloro-1-cyclopropyl-7-fluoro-4-oxo-1,4-dihydro-benzo[b][1,8]naphtyridine-3-carboxylate d'éthyle et de 6 g de 4-(4-fluoroanilino) pipéridine dans 80 cm³ de diméthylsulfoxyde est chauffée, sous agitation, à une température voisine de 110°C pendant environ 10 heures. Après refroidissement vers 20°C, le mélange réactionnel est concentré sous pression réduite (20 kPa) à une température voisine de 140°C. Le résidu noir est recristallisé dans un mélange de 75 cm³ d'éthanol et 35 cm³ de diméthylformamide On obtient 2,35 g de 1-cyclopropyl-7-fluoro-8-[4-(-4-fluoroanilino)-pipéridino-4-oxo-1,4-dihydro-benzo[b][1,8]naphtyridine-3-carboxylate d'éthyle sous forme d'un solide brun fondant vers 230°C qui est utilisé sans autre purification pour les étapes ultérieures.

Le 8-chloro-1-cyclopropyl-7-fluoro-4-oxo-1,4-dihydro-benzo[b][1,8]naphtyridine-3-carboxylate d'éthyle est décrit dans le brevet EP 431 991.

### Exemple 16

### Acide 1-(2,4-difluorophényl)-7-fluoro-8-[4-(4-fluorophényl)-pipérazin-1-yl]-4-oxo-1,4-dihydro-benzo[b][1,8]naphtyridine-3-carboxylique :

L'acide 1-(2,4-difluorophényl)-7-fluoro-8-[4-(4-fluorophényl)-pipérazin-1-yl]-4-oxo-1,4-dihydro-benzo[b][1,8]naphtyridine-3-carboxylique est préparé dans les conditions de l'exemple 1, mais à partir de 1,55 g de l'acide-7,8-difluoro-1-(2,4-difluorophényl)-4-oxo-1,4-dihydro-benzo[b][1,8]naphtyridine-3-carboxylique et de 1,58 g de 4-(4-fluorophényl) pipérazine dans 15 cm³ de diméthylsulfoxyde. Après 1 recristallisation dans 55 cm³ de diméthylformamide, on obtient 1,4 g de l'acide 1-(2,4-difluorophényl)-7-fluoro-8-[4-(4-fluorophényl)-pipérazin-1-yl]-4-oxo-1,4-dihydro-benzo[b][1,8]naphtyridine-3-carboxylique sous forme d'un solide jaune fondant à 322°C.

L'acide 7,8-difluoro-1-(2,4-difluorophényl)-4-oxo-1,4-dihydro-benzo[b][1,8] naphtyridine-3-carboxylique a été obtenu dans les conditions décrites à l'exemple 3, mais à partir de 3,2 g du 1-(2,4-difluorophényl)-7-fluoro-8-[4-(4-fluorophényl)-pipérazin-1-yl]-4-oxo-1,4-dihydro-benzo[b][1,8]naphtyridine-3 carboxylate d'éthyle dans 32 cm³ d'acide acétique et 32 cm³ d' acide chlorhydrique en solution aqueuse à 17,5%. On obtient 2,7 g de l'acide 7,8-difluoro-1-(2,4-difluorophényl)-4-oxo-1,4-dihydro-benzo[b][1,8]naphtyridine-3-carboxylique sous forme d'un solide jaune fondant à 318-320°C.

Le 1-(2,4-difluorophényl)-7-fluoro-8-[4-(4-fluorophényl)-pipérazin-1-yl]-4-oxo-1,4-dihydro-benzo[b][1,8]naphtyridine-3 carboxylate d'éthyle dans les conditions de l'exemple 13, mais à partir de 3,68 g de 3-(2-chloro-6,7-difluoro-3-quinolyl)-2-diméthylamino-3-oxo-propionate d'éthyle et de 3,87 g de 2,4-difluoro aniline dans 40 cm³ de trichlorométhane Le mélange est additionné de 50 cm³ d'éthanol et de 1,5 cm³ de 1,8-diazabicyclo[5.4.0]undec-7-ène et chauffé à une température voisine de 80°C pendant environ 1 heure. Après refroidissement à une température voisine de 10°C, le produit est essoré, lavé par 2 fois 50 cm³ d'éthanol et 2 fois 50 cm³ d'éther isopropylique. On obtient 3,3 g de-1-(2,4-difluorophényl)-7,8-difluoro-4-oxo-1,4-dihydro-benzo[b][1,8]naphtyridine-3-carboxylate d'éthyle sous forme d'un solide jaune fondant vers 300°C qui est utilisé sans autre purification pour les étapes ultérieures.

Le 3-(2-chloro-6,7-difluoro-3-quinolyl)-2-diméthylaminométhylène-3-oxo-propionate d'éthyle est décrit dans le brevet EP 431 991.

### Exemple 17

### Acide-8-(cyclohexyl éthyl amino)-7-fluoro-1-méthyl-4-oxo-1,4-dihydro-benzo[b] [1,8] naphtyridine-3-carboxylique :

L'acide-8-(cyclohexyl éthyl amino)-7-fluoro-1-méthyl-4-oxo-1,4-dihydro-benzo[b] [1,8]naphtyridine-3-carboxylique est préparé à partir de 0,5 g de 8-cyclohexylamino-7-fluoro-1-méthyl-4-oxo-1,4-dihydro-benzo[b][1,8]naphtyridine-3-carboxylate d'éthyle, 0,19 cm³ de iodoéthane, 0,096 g d'hydrure de sodium dispersé à 60 % dans l'huile minérale et 10 cm³ de diméthylformamide. Après traitement avec 10 cm³ d'eau et 2 cm³ d'acide chlorhydrique 1 N, le mélange est extrait par 3 fois 20 cm³ de trichlorométhane. Les phases organiques réunies sont lavées par 1 fois 20 cm³ d'eau, séchées sur sulfate de magnésium et concentrées sous pression réduite (20 kPa) à une température voisine de 40°C. On obtient 0,25 g de l'acide-8-(cyclohexyl éthyl amino)-7-fluoro-1-méthyl-4-oxo-1,4-dihydro-benzo[b][1,8]naphtyridine-3-carboxylique. sous forme d'un solide jaune fondant à 230°C.

Le 8-cyclohexylamino-7-fluoro-1-méthyl-4-oxo-1,4-dihydro-benzo[b][1,8]naphtyridine-3-carboxylate d'éthyle a été préparé dans les conditions de l'exemple 4, mais à partir de 3 g de 7,8-difluoro-1-méthyl-4-oxo-1,4-dihydro-benzo[b][1,8]naphty-ridine-3-carboxylate d'éthyle et de 2,2 cm³ de cyclohexylamine dans 30 cm³ de diméthylsulfoxyde. Après un essorage avec 15 cm³ d'éthanol par 2 fois, on obtient 3,3 g de-8-cyclohexyl-7-fluoro-1-méthyl-4-oxo-1,4-dihydro-benzo[b][1,8]naphtyridine-3-carboxylate d'éthyle sous forme d'un solide jaune fondant à 220°C.

La présente invention concerne également les compositions pharmaceutiques utilisables en médecine humaine ou vétérinaire qui contiennent comme produit actif au moins un produit de formule générale (I) à l'état pur (sous forme libre ou sous forme de sel) ou sous forme d'une association avec un ou plusieurs diluants ou adjuvants compatibles et pharmaceutiquement acceptables. Ces compositions peuvent être utilisées par voie topique.

Les compositions pour administration topique peuvent être par exemple des crèmes, des pommades, des gels, des lotions, des liniments ou des aérosols il peut s'agir également de compositions solides pulvérulentes. Elles peuvent être destinées notamment à l'administration nasale.

Lorsque les compositions sont des crèmes, des pommades ou des gels, ces compositions peuvent être par exemple des pommades hydrophiles contenant par exemple des polyéthylèneglycols et des quantités appropriées d'eau, des pommades hydrophobes contenant par exemple de la vaseline, de la paraffine, de la paraffine liquide, des huiles végétales ou des graisses animales, des glycérides synthétiques, des cires ou des polyalkylsiloxanes liquides, elles peuvent être également des crèmes hydrophiles contenant notamment des agents émulsifiants huile dans eau comme par exemple des savons de sodium ou de triéthanolamine, des alcools gras, des alcools gras sulfatés, des polysorbates en combinaison éventuellement avec des agents émulsifiants eau dans huile, ou des crèmes hydrophobes contenant notamment des agents émulsifiants eau dans huile tels que la graisse de laine, les esters de sorbitannes, les monoglycérides, elles peuvent être aussi des gels hydrophiles à base d'eau, d'alcool, de glycerol ou de propylèneglycol gélifiés, ou des gels hydrophobes comprenant de la paraffine liquide additionnée de polyéthylène, des huiles grasses gélifiées par l'oxyde de silicium colloïdal ou des savons d'aluminium ou de zinc.

A titre d'exemple, lorsque les compositions sont des aérosols, pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable ; pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine ou vétérinaire, les compositions selon l'invention sont particulièrement utiles dans le traitement prophylactique ou curatif des infections cutanéo-muqueuses d'origine bactérienne à Gram-positif, en particulier traitement curatif des infections cutanées à bactéries Gram positif et/ou traitement préventif des infections à bactéries Gram positif multirésistantes. Notamment dans le traitement des infections associées à des plaies, des greffes ou des brûlures, dans le traitement des infections liées aux lésions de la peau, ou dans le traitement des impétigos et des furonculoses, ainsi également que pour la prévention de la contamination des voies nasales par les bactéries Gram-positif multirésistantes, et aussi pour la décontamination en vue d'éviter la dissémination de ces germes.

D'une façon générale, le médecin déterminera la posologie qu'il estime la plus appropriée en fonction de l'âge, du degré de l'infection et des autres facteurs propres au sujet à traiter. Généralement, le principe actif est contenu à 1 ou 2 % dans la formulation. Cette formulation est appliquée 1 à 3 fois par jour par voie topique, de préférence 1 ou 2 fois par jour.

L'exemple suivant, donné à titre non limitatif, illustre une composition selon l'invention :

### Exemple

On prépare selon les techniques habituelles une crème dosée à 2 % d'acide libre, ayant la composition suivante :
- 7-fluoro-8-[4-(4-fluorophényl)-pipérazin-1-yl]-1-métyl-4-oxo-1,4-dihydro-benzo[b][1,8]naphtyridine-3-carboxylate de choline 2,52 mg
- cetomacrogol 30,0 mg
- alcool benzylique 3,0 mg
- eau ppi 100 mg

Par ailleurs, les produits de formule générale (I) peuvent également être utilisés comme agents de conservation ou de désinfection des matières organiques ou minérales. Notamment dans l'industrie des colorants, de matières grasses, du papier, du bois, des polymères ou encore dans l'industrie textile, l'industrie alimentaire ou le traitement des eaux. Il est également entendu que les compositions renfermant un produit de formule générale (I) à l'état pur ou sous forme d'association avec des diluants ou adjuvants compatibles, entrent aussi dans le cadre de la présente invention.

## Revendications

1. Un dérivé de la benzo[b]naphtyridine-1,8 **caractérisé en ce qu'**il répond à la formule générale : 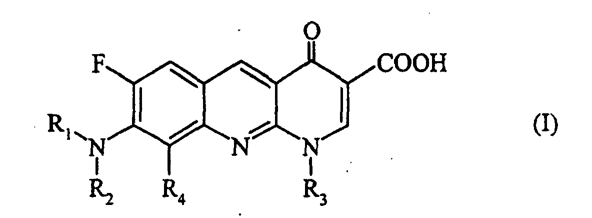 dans laquelle,
- R₁ et R₂ identiques ou différents représentent un radical alcoyle ou cycloalcoyle contenant 3 à 8 chaînons, ou forment ensemble avec l'atome d'azote auquel ils sont attachés un hétérocycle à 6 chaînons contenant éventuellement un autre atome d'azote et pouvant être substitué en position -4 par un radical phényle, phényle substitué (par un atome d'halogène ou par un radical alcoylthiométhyle), benzyle, benzyle substitué par un atome d'halogène, hétérocyclylméthyle dont la partie hétérocyclyle est saturée ou insaturée et contient 5 ou 6 chaînons, phénylamino, ou phénylamino dont la partie phényle est éventuellement substituée par un atome d'halogène, ou R₁ et R₂ forment ensemble avec l'atome d'azote auquel ils sont attachés un hétérocycle à 7 ou 8 chaînons contenant éventuellement un autre atome d'azote et pouvant être substitué par l'un des substituants de l'hétérocycle à 6 chaînons énumérés ci-dessus,
- R₃ représente un radical alcoyle, fluoroalcoyle, carboxyalcoyle, cycloalcoyle contenant 3 à 6 atomes de carbone, fluorophényle, difluorophényle, alcoyloxy ou alcoylamino, et
- R₄ représente un atome d'hydrogène ou un atome de fluor,
les radicaux alcoyle cités ci-dessus étant droits ou ramifiés et contenant 1 à 4 atomes de carbone,
le cas échéant sous ses formes stéréoisomères ou leurs mélanges, ainsi que ses sels métalliques, ses sels d'addition avec les bases azotées et ses sels d'addition avec les acides.

2. Un dérivé de la benzo[b]naphtyridine-1,8 selon la revendication 1, **caractérisé en ce que** :
- R₁ et R₂ identiques ou différents représentent un radical alcoyle contenant 1 ou 2 atomes de carbone, ou cyclopropyle, ou forment ensemble avec l'atome d'azote auquel ils sont attachés un hétérocycle à 6 chaînons, contenant éventuellement un autre atome d'azote et pouvant être substitué en position -4 par un radical phényle, phényle substitué par un atome d'halogène ou par un radical méthylthiométhyle, benzyle, benzyle substitué par un atome d'halogène, hétérocyclylméthyle dont la partie hétérocyclyle est insaturée et contient 5 chaînons, phénylamino dont la partie phényle est éventuellement substituée par un atome d'halogène, ou R₁ et R₂ forment ensemble avec l'atome d'azote auquel ils sont attachés un hétérocycle à 7 ou 8 chaînons,
- R₃ représente un radical alcoyle contenant 1 ou 2 atomes de carbone, cyclopropyle, difluorophényle ou méthylamino, et
- R₄ représente un atome d'hydrogène ou un atome de fluor
le cas échéant sous ses formes stéréoisomères ou leurs mélanges, ainsi que ses sels métalliques, ses sels d'addition avec les bases azotées et ses sels d'addition avec les acides.

3. Un dérivé de la benzo[b]naphtyridine-1,8 selon la revendication 1, **caractérisé en ce qu'**il s'agit de l'acide-7-fluoro-8-[4-(4-fluorophényl)-pipérazin-1-yl]-1-méthyl-4-oxo-1,4-dihydro-benzo[b][1,8]naphtyridine-3-carboxylique et ses sels d'addition avec les acides.

4. Procédé de préparation d'un dérivé de la benzo[b]naphtyridine-1,8 selon la revendication 1, **caractérisé en ce que** l'on fait agir une amine de formule générale :
R₁-NH-R₂
dans laquelle R₁ et R₂ sont définis comme dans la revendication 1, sur une benzo[b]naphtyridine-1,8 de formule générale : 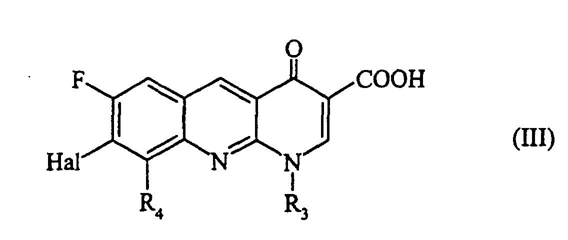 dans laquelle R₃ et R₄ sont définis comme dans la revendication 1 et Hal est un atome de fluor, de chlore ou de brome, si R₄ est un atome d'hydrogène, ou Hal et R₄ sont simultanément des atomes de fluor, puis éventuellement transforme le produit obtenu en un sel.

5. Procédé de préparation d'un dérivé de la benzo[b]naphtyridine-1,8 selon la revendication 1, **caractérisé en ce que** l'on transforme l'ester de formule générale : 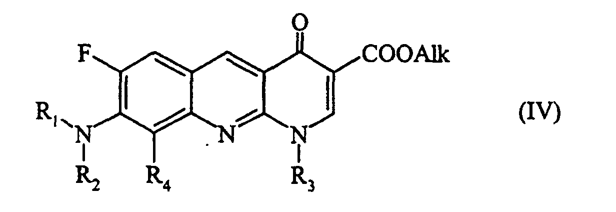 dans laquelle R₁, R₂ et R₄ sont définis comme dans la revendication 1, R₃ est défini comme dans la revendication 1 ou représente un radical alcoylamino protégé et Alk est un radical alcoyle droit ou ramifié contenant 1 à 4 atomes de carbone, par toute méthode connue pour obtenir un acide à partir d'un ester, sans toucher au reste de la molécule, puis, le cas échéant, élimine le groupement protecteur du radical alcoylamino, et/ou éventuellement prépare le sel du dérivé de benzo[b]naphtyridine obtenu.

6. Composition **caractérisée en ce qu'**elle contient au moins un dérivé selon l'une des revendications 1 à 3, à l'état pur ou en association avec un ou plusieurs diluants ou adjuvants compatibles.

7. Composition pharmaceutique selon la revendication 6, **caractérisée en ce qu'**elle contient au moins un dérivé selon l'une des revendications 1 à 3, à l'état pur ou en association avec un ou plusieurs diluants ou adjuvants compatibles et pharmaceutiquement acceptable.

## Claims

1. Benzo[b][1,8]naphthyridine derivative, **characterized in that** it corresponds to the general formula: 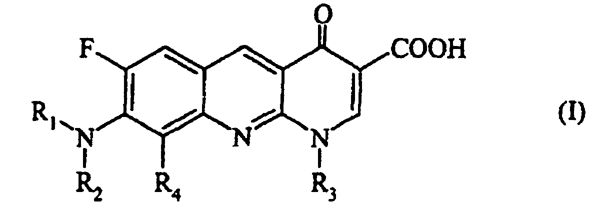 in which:
- R₁ and R₂, which are identical or different, represent an alkyl or cycloalkyl radical comprising 3 to 8 members or form, together with the nitrogen atom to which they are attached, a 6-membered heterocycle optionally comprising another nitrogen atom which can be substituted at the 4-position by a phenyl radical, a substituted phenyl radical (substituted by a halogen atom or by an alkylthiomethyl radical), a benzyl radical, a benzyl radical substituted by a halogen atom, a heterocyclylmethyl radical, the heterocyclyl part of which is saturated or unsaturated and comprises 5 or 6 members, a phenylamino radical or a phenylamino radical, the phenyl part of which is optionally substituted by a halogen atom, or R₁ and R₂ form, together with the nitrogen atom to which they are attached, a 7- or 8-membered heterocycle optionally comprising another nitrogen atom which can be substituted by one of the substituents of the 6-membered heterocycle which are listed above,
- R₃ represents an alkyl radical, a fluoroalkyl radical, a carboxyalkyl radical, a cycloalkyl radical comprising 3 to 6 carbon atoms, a fluorophenyl radical, a difluorophenyl radical, an alkyloxy radical or an alkylamino radical, and
- R₄ represents a hydrogen atom or a fluorine atom, the abovementioned alkyl radicals being straight or branched and comprising 1 to 4 carbon atoms, if appropriate in its stereoisomeric forms or their mixtures, as well as its metal salts, its addition salts with nitrogenous bases and its addition salts with acids.

2. Benzo[b][1,8]naphthyridine derivative according to Claim 1, **characterized in that**:
- R₁ and R₂, which are identical or different, represent an alkyl radical comprising 1 or 2 carbon atoms or a cyclopropyl radical or form, together with the nitrogen atom to which they are attached, a 6-membered heterocycle optionally comprising another nitrogen atom which can be substituted at the 4-position by a phenyl radical, a phenyl radical substituted by a halogen atom or by a methylthiomethyl radical, a benzyl radical, a benzyl radical substituted by a halogen atom, a heterocyclylmethyl radical, the heterocyclyl part of which is unsaturated and comprises 5 members, or a phenylamino radical, the phenyl part of which is optionally substituted by a halogen atom, or R₁ and R₂ form, together with the nitrogen atom to which they are attached, a 7- or 8-membered heterocycle,
- R₃ represents an alkyl radical comprising 1 or 2 carbon atoms, a cyclopropyl radical, a difluorophenyl radical or a methylamino radical, and
- R₄ represents a hydrogen atom or a fluorine atom, if appropriate in its stereoisomeric forms or their mixtures, as well as its metal salts, its addition salts with nitrogenous bases and its addition salts with acids.

3. Benzo[b][1,8]naphthyridine derivative according to Claim 1, **characterized in that** it is 7-fluoro-8-[4-(4-fluorophenyl)piperazin-1-yl]-1-methyl-4-oxo-1,4-dihydrobenzo[b][1,8]naphthyridine-3-carboxylic acid and its addition salts with acids.

4. Process for the preparation of a benzo[b][1,8]naphthyridine derivative according to Claim 1, **characterized in that** an amine of general formula:
R₁-NH-R₂
in which R₁ and R₂ are defined as in Claim 1, is reacted with a benzo[b][1,8]naphthyridine of general formula: 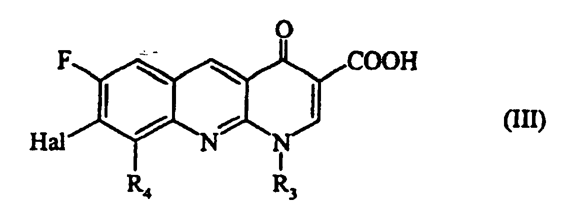 in which R₃ and R₄ are defined as in Claim 1 and Hal is a fluorine, chlorine or bromine atom if R₄ is a hydrogen atom, or Hal and R₄ are simultaneously fluorine atoms, and then the product obtained is optionally converted to a salt.

5. Process for the preparation of a benzo[b][1,8]naphthyridine derivative according to Claim 1, **characterized in that** the ester of general formula: 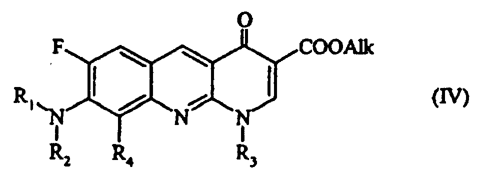 in which R₁, R₂ and R₄ are defined as in Claim 1, R₃ is defined as in Claim 1 or represents a protected alkylamino radical, and Alk is a straight or branched alkyl radical comprising 1 to 4 carbon atoms, is converted by any method known for producing an acid from an ester without affecting the remainder of the molecule, then, if appropriate, the protecting group is removed from the alkylamino radical and/or, optionally, the salt of the benzo[b]naphthyridine derivative obtained is prepared.

6. Composition, **characterized in that** it comprises at least one derivative according to one of Claims 1 to 3, in the pure state or in combination with one or more compatible diluents or adjuvants.

7. Pharmaceutical composition according to Claim 6, **characterized in that** it comprises at least one derivative according to one of Claims 1 to 3, in the pure state or in combination with one or more compatible and pharmaceutically acceptable diluents or adjuvants.

## Patentansprüche

1. Ein Derivat von Benzo[b]-1,8-naphthyridin, **dadurch gekennzeichnet, dass** es der allgemeinen Formel: 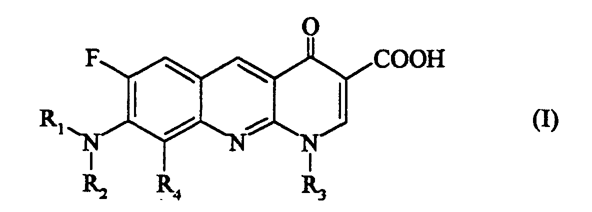 entspricht, worin
- R₁ und R₂, die gleich oder verschieden sind, einen Alkyl- oder Cycloalkylrest mit 3 bis 8 Ringgliedern darstellen oder zusammen mit dem Stickstoffatom, an das sie gebunden sind, einen Heterocyclus mit 6 Ringgliedern bilden, der gegebenenfalls ein weiteres Stickstoffatom enthält und in Position 4 substituiert sein kann mit einem Rest Phenyl, substituiertes Phenyl (mit einem Halogenatom oder mit einem Alkylthiomethylrest), Benzyl, mit einem Halogenatom substituiertes Benzyl, Heterocyclylmethyl, dessen Heterocyclylteil gesättigt oder ungesättigt ist und 5 oder 6 Ringglieder enthält, Phenylamino oder Phenylamino, dessen Phenylteil gegebenenfalls mit einem Halogenatom substituiert ist, oder R₁ und R₂ zusammen mit dem Stickstoffatom, an das sie gebunden sind, einen Heterocyclus mit 7 oder 8 Ringgliedern bilden, der gegebenenfalls ein weiteres Stickstoffatom enthält und der mit einem der oben aufgezählten Substituenten des Heterocyclus mit 6 Ringgliedern substituiert sein kann,
- R₃ einen Rest Alkyl, Fluoralkyl, Carboxyalkyl, Cycloalkyl mit 3 bis 6 Kohlenstoffatomen, Fluorphenyl, Difluorphenyl, Alkyloxy oder Alkylamino darstellt und
- R₄ ein Wasserstoffatom oder ein Fluoratom darstellt,
wobei die oben angeführten Alkylreste gerade oder verzweigt sind und 1 bis 4 Kohlenstoffatome enthalten,
gegebenenfalls in seinen stereoisomeren Formen oder deren Gemische sowie seine Metallsalze, seine Additionssalze mit den Stickstoffbasen und seine Additionssalze mit den Säuren.

2. Ein Derivat von Benzo[b]-1,8-naphthyridin gemäß Anspruch 1, **dadurch gekennzeichnet, dass**:
- R₁ und R₂, die gleich oder verschieden sind, einen Alkylrest mit 1 oder 2 Kohlenstoffatomen oder den Cyclopropylrest darstellen oder zusammen mit dem Stickstoffatom, an das sie gebunden sind, einen Heterocyclus mit 6 Ringgliedern bilden, der gegebenenfalls ein weiteres Stickstoffatom enthält und in Position 4 substituiert sein kann mit einem Rest Phenyl, mit einem Halogenatom oder mit einem Methylthiomethylrest substituiertes Phenyl, Benzyl, mit einem Halogenatom substituiertes Benzyl, Heterocyclylmethyl, dessen Heterocyclylteil ungesättigt ist und 5 Ringglieder enthält, Phenylamino, dessen Phenylteil gegebenenfalls mit einem Halogenatom substituiert ist, oder R₁ und R₂ zusammen mit dem Stickstoffatom, an das sie gebunden sind, einen Heterocyclus mit 7 oder 8 Ringgliedern bilden,
- R₃ einen Alkylrest mit 1 oder 2 Kohlenstoffatomen, einen Rest Cyclopropyl, Difluorphenyl oder Methylamino darstellt und
- R₄ ein Wasserstoffatom oder ein Fluoratom darstellt,
gegebenenfalls in seinen stereoisomeren Formen oder deren Gemische sowie seine Metallsalze, seine Additionssalze mit den Stickstoffbasen und seine Additionssalze mit den Säuren.

3. Ein Derivat von Benzo[b]-1,8-naphthyridin gemäß Anspruch 1, **dadurch gekennzeichnet, dass** es sich um 7-Fluor-8-[4-(4-fluorphenyl)-piperazin-1-yl]-1-methyl-4-oxo-1,4-dihydro-benzo[b][1,8]naphthyridin-3-carbonsäure und ihre Additionssalze mit den Säuren handelt.

4. Verfahren zur Herstellung eines Derivats von Benzo[b]-1,8-naphtyridin gemäß Anspruch 1, **dadurch gekennzeichnet, dass** man ein Amin der allgemeinen Formel:
R₁-NH-R₂
worin R₁ und R₂ wie in Anspruch 1 definiert sind, mit einem Benzo[b]-1,8-naphthyridin der allgemeinen Formel: 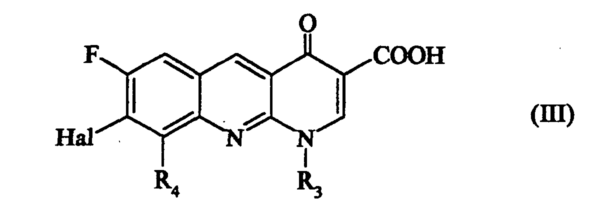 worin R₃ und R₄ wie in Anspruch 1 definiert sind und Hal ein Fluor-, Chlor- oder Bromatom ist, wenn R₄ ein Wasserstoffatom ist, oder Hal und R₄ gleichzeitig Fluoratome sind, umsetzt, dann das erhaltene Produkt gegebenenfalls in ein Salz überführt.

5. Verfahren zur Herstellung eines Derivats von Benzo[b]-1,8-naphtyridin gemäß Anspruch 1, **dadurch gekennzeichnet, dass** man den Ester der allgemeinen Formel: 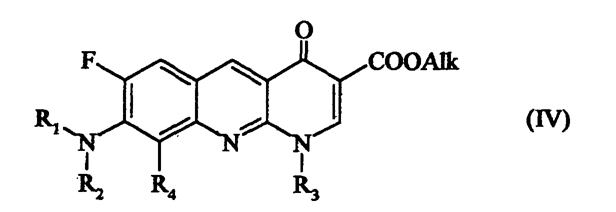 worin R₁, R₂ und R₄ wie in Anspruch 1 definiert sind, R₃ wie in Anspruch 1 definiert ist oder einen geschützten Alkylaminorest darstellt und Alk ein gerader oder verzweigter Alkylrest mit 1 bis 4 Kohlenstoffatomen ist, durch jedes bekannte Verfahren zum Erhalt einer Säure aus einem Ester umwandelt, ohne den Rest des Moleküls zu berühren, dann gegebenenfalls die Schutzgruppe des Alkylaminorests entfernt und/oder gegebenenfalls das Salz des erhaltenen Benzo[b]naphthyridinderivats herstellt.

6. Zusammensetzung, **dadurch gekennzeichnet, dass** sie wenigstens ein Derivat gemäß einem der Ansprüche 1 bis 3 in reinem Zustand oder in Kombination mit einem oder mehreren kompatiblen Verdünnungsmitteln oder Zusatzstoffen enthält.

7. Pharmazeutische Zusammensetzung gemäß Anspruch 6, **dadurch gekennzeichnet, dass** sie wenigstens ein Derivat gemäß einem der Ansprüche 1 bis 3 in reinem Zustand oder in Kombination mit einem oder mehreren kompatiblen und pharmazeutisch annehmbaren Verdünnungsmitteln oder Zusatzstoffen enthält.
